(19) Europäisches Patentamt
European Patent Office
Office européen des brevets

(11) **EP 4 425 490 A1**

(12) **EUROPEAN PATENT APPLICATION**
published in accordance with Art. 153(4) EPC

(43) Date of publication:
**04.09.2024   Bulletin 2024/36**

(21) Application number: **22886921.0**

(22) Date of filing: **24.10.2022**

(51) International Patent Classification (IPC):
***G10L 21/0364*** [(2013.01)]

(52) Cooperative Patent Classification (CPC):
**A61B 5/374; A61B 5/38; A61B 5/486; A61M 21/00;
G10H 1/057; G10H 5/10; G10K 15/02;
G10L 21/0364; G16H 20/70; G16H 40/63;
A61M 2021/0027; A61M 2021/005;
A61M 2205/3584; A61M 2205/505; A61M 2205/581;**

(Cont.)

(86) International application number:
**PCT/JP2022/039422**

(87) International publication number:
**WO 2023/074594 (04.05.2023 Gazette 2023/18)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **25.10.2021   JP 2021173634
09.05.2022   JP 2022077088**

(72) Inventors:
• **NAGATANI Yoshiki**
**Tokyo 104-0028 (JP)**
• **TAKAZAWA Kazuki**
**Tokyo 104-0028 (JP)**
• **OGAWA Koichi**
**Osaka-shi, Osaka 541-0045 (JP)**
• **MAEDA Kazuma**
**Osaka-shi, Osaka 541-0045 (JP)**
• **YANAGAWA Tatsuya**
**Osaka-shi, Osaka 541-0045 (JP)**

(74) Representative: **Plasseraud IP**
**104 Rue de Richelieu**
**CS92104**
**75080 Paris Cedex 02 (FR)**

(71) Applicants:
• **Pixie Dust Technologies, Inc.**
**Tokyo, 104-0028 (JP)**
• **Shionogi & Co., Ltd**
**Osaka-shi, Osaka 541-0045 (JP)**

(54) **SIGNAL PROCESSING DEVICE, COGNITIVE FUNCTION IMPROVEMENT SYSTEM, SIGNAL PROCESSING METHOD, AND PROGRAM**

(57)   The signal processing apparatus has a means for receiving an input acoustic signal, and amplitude-modulates the received input acoustic signal to have an amplitude change corresponding to the frequency of the gamma wave, and the rise and fall of the envelope of the amplitude waveform. Means for generating an output acoustic signal that is asymmetric and means for outputting the generated output acoustic signal.

[FIG. 3]

EP 4 425 490 A1

**(Cont. next page)**

(52) Cooperative Patent Classification (CPC): (Cont.)
A61M 2230/10; G10H 2220/376; G16H 10/20

**Description**

[Technical Field]

The present disclosure relates to a signal processing apparatus, a signal processing method, and a program.

[Background Art]

There is a research report that when an organism is made to perceive a pulsed sound stimulus at a frequency of about 40 times per second to induce gamma waves in the brain of the organism, it is effective in improving the cognitive function of the organism (non-patent literature). 1). Gamma waves refer to those whose frequency is included in the gamma band (25 to 140 Hz) among nerve vibrations obtained by capturing periodic nerve activity in the cortex of the brain by electrophysiological techniques such as electroencephalograms and magnetoencephalography.
Patent Document 1 discloses adjusting the volume by increasing or decreasing the amplitude of sound waves or soundtracks to create rhythmic stimulation corresponding to stimulation frequencies that induce brain wave entrainment.

[Prior Art Documents]

[Patent Documents]

[Patent Document 1] Japanese Patent Publication No. 2020-501853

[Non-Patent Documents]

[Non-Patent Document 1] Multi-sensory Gamma Stimulation Ameliorates Alzheimer's-Associated Pathology and Improves Cognition Cell 2019 Apr 4;177(2):256-271.e22. doi: 10.1016/j.cell.2019.02.014.

[Summary of Invention]

[Technical Problem]

However, sufficient consideration has not been made as to what kind of waveform is desirable as the amplitude waveform when the amplitude of the acoustic signal is increased or decreased. Even for acoustic signals having the same frequency and periodicity, it is conceivable that the magnitude of the cognitive function improvement effect and the discomfort given to the listener differ depending on the characteristics of the amplitude waveform.
An object of the present disclosure is to change the amplitude of an acoustic signal while suppressing discomfort given to the listener.

[Solution to Problem]

A signal processing apparatus according to one aspect of the present disclosure includes means for receiving an input acoustic signal, and amplitude-modulating the received input acoustic signal to have an amplitude change corresponding to a gamma wave frequency, and an amplitude waveform envelope means for generating an output acoustic signal whose rise and fall are asymmetric; and means for outputting the generated output acoustic signal.

[Brief Description of Drawings]

FIG. 1 is a block diagram showing the configuration of an acoustic system according to this embodiment;
FIG. 2 is a block diagram showing the configuration of a signal processing apparatus according to the embodiment;
FIG. 3 is an explanatory diagram of one aspect of the present embodiment;
FIG. 4 is a diagram showing a first example of an amplitude waveform of an output acoustic signal;
FIG. 5 is a diagram showing a second example of an amplitude waveform of an output acoustic signal;
FIG. 6 is a diagram showing a third example of an amplitude waveform of an output acoustic signal;
FIG. 7 is a diagram showing experimental results;
FIG. 8 is a diagram showing the overall flow of acoustic signal processing by the signal processing apparatus of the present embodiment;

FIG. 9 is a diagram showing a list of sound stimuli used in experiments;

FIG. 10 is a diagram showing experimental results of electroencephalogram evoked by sound stimulation; and

FIG. 11 is a diagram showing the correlation between results of psychological experiments and electroencephalogram measurements.

[Description of Embodiments]

Hereinafter, an embodiment of the present invention is described in detail based on the drawings. Note that, in the drawings for describing the embodiments, the same components are denoted by the same reference sign in principle, and the repetitive description thereof is omitted.

(1) Acoustic system configuration

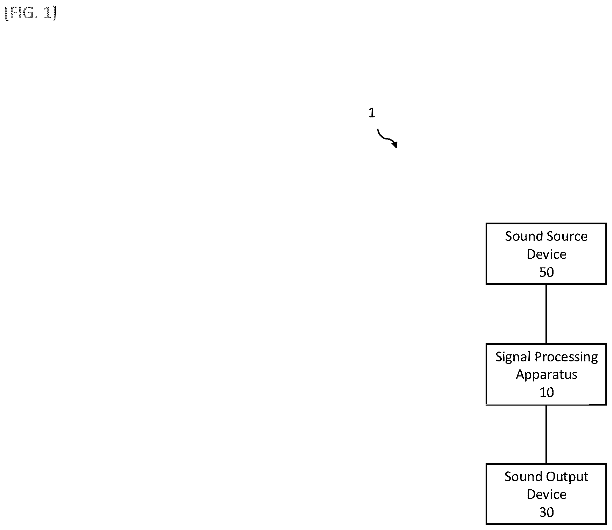

The configuration of the acoustic system will be explained. FIG. 1 is a block diagram showing the configuration of the acoustic system of this embodiment.

As shown in FIG. 1, the acoustic system 1 includes a signal processing apparatus 10, a sound output device 30, and a sound source device 50.

The signal processing apparatus 10 and the sound source device 50 are connected to each other via a predetermined interface capable of transmitting acoustic signals. The interface is, for example, SPDIF (Sony Philips Digital Interface), HDMI (registered trademark) (High-Definition Multimedia Interface), a pin connector (RCA pin), or an audio interface for headphones. The interface may be a wireless interface using Bluetooth (registered trademark) or the like. The signal processing apparatus 10 and the sound output device 30 are similarly connected to each other via a predetermined interface. The acoustic signal in this embodiment includes either or both of an analog signal and a digital signal.

The signal processing apparatus 10 performs acoustic signal processing on the input acoustic signal acquired from the sound source device 50 . Acoustic signal processing by the signal processing apparatus 10 includes at least modulation processing of an acoustic signal (details will be described later). Also, the acoustic signal processing by the signal processing apparatus 10 may include conversion processing (for example, separation, extraction, or synthesis) of acoustic signals. Furthermore, the acoustic signal processing by the signal processing apparatus 10 may further include acoustic signal amplification processing similar to that of an AV amplifier, for example. The signal processing apparatus 10 sends the output acoustic signal generated by the acoustic signal processing to the sound output device 30 . The signal processing apparatus 10 is an example of an information processing apparatus.

The sound output device 30 generates sound according to the output acoustic signal acquired from the signal processing apparatus 10 . The sound output device 30 may include, for example, a loudspeaker (amplified speaker (powered speaker)). ), headphones, or earphones.

The sound output device 30 can also be configured as one device together with the signal processing apparatus 10 . Specifically, the signal processing apparatus 10 and the sound output device 30 can be implemented as a TV, radio, music player, AV amplifier, speaker, headphone, earphone, smart phone, or PC. The signal processing apparatus 10 and the sound output device 30 constitute a cognitive function improvement system.

Sound source device 50 sends an input acoustic signal to signal processing apparatus 10 . The sound source device 50 is, for example, a TV, a radio, a music player, a smart phone, a PC, an electronic musical instrument, a telephone, a video game console, a game machine, or a device that conveys an acoustic signal by broadcasting or information communication.

(1-1) Configuration of signal processing apparatus

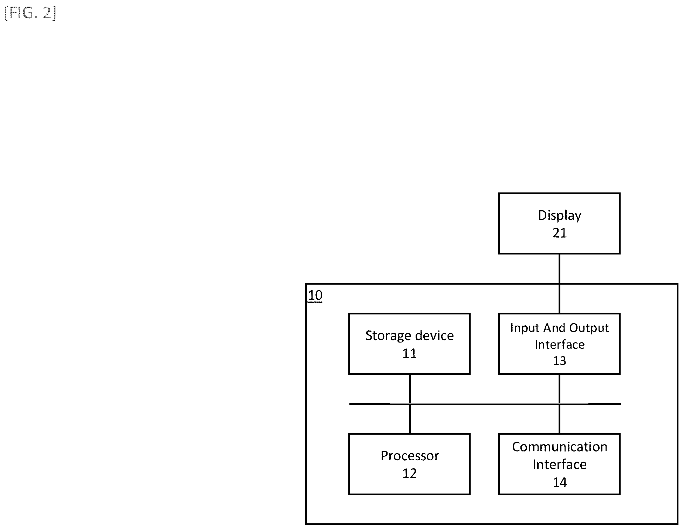

A configuration of the signal processing apparatus will be described. FIG. 2 is a block diagram showing the configuration of the signal processing apparatus of this embodiment.

As shown in FIG. 2 , the signal processing apparatus 10 includes a storage device 11 , a processor 12, an input/output interface 13, and a communication interface 14 . The signal processing apparatus 10 is connected to the display 21 .

The memory 11 is configured to store a program and data. The memory 11 is, for example, a combination of a ROM (read only memory), a RAM (random access memory), and a storage (for example, a flash memory or a hard disk). The program and data may be provided via a network, or may be provided by being recorded on a computer-readable recording medium.

Programs include, for example, the following programs:

OS (Operating System) program; and

program of an application that executes an information processing.

The data includes, for example, the following data:

Databases referenced in information processing; and
· Data obtained by executing an information processing (that is, an execution result of an information processing).

The processor 12 is a computer that implements the functions of the signal processing apparatus 10 by reading and executing programs stored in the storage device 11 . At least part of the functions of the signal processing apparatus 10 may be realized by one or more dedicated circuits. Processor 12 is, for example, at least one of the following: CPU (Central Processing Unit);

GPU (Graphic Processing Unit);
ASIC (Application Specific Integrated Circuit);
FPGA (Field Programmable Array); and
DSP (digital signal processor).

The input/output interface 13 is configured to acquire user instructions from input devices connected to the signal processing apparatus 10 and to output information to output devices connected to the signal processing apparatus 10 .

The input device is, for example, the sound source device 50, physical buttons, keyboard, a pointing device, a touch panel, or a combination thereof.

The output device is, for example, display 21, sound output device 30, or a combination thereof.

Further, the input/output interface 13 may include signal processing hardware such as A/D converters, D/A converters, amplifiers, mixers, filters, and the like.

The communication interface 14 is configured to control communication between the signal processing apparatus 10 and an external device (e.g., the sound output device 30 or the sound source device 50).

The display 21 is configured to display images (still images or moving images). The display 21 is, for example, a liquid crystal display or an organic EL display.

(2) One aspect of the embodiment

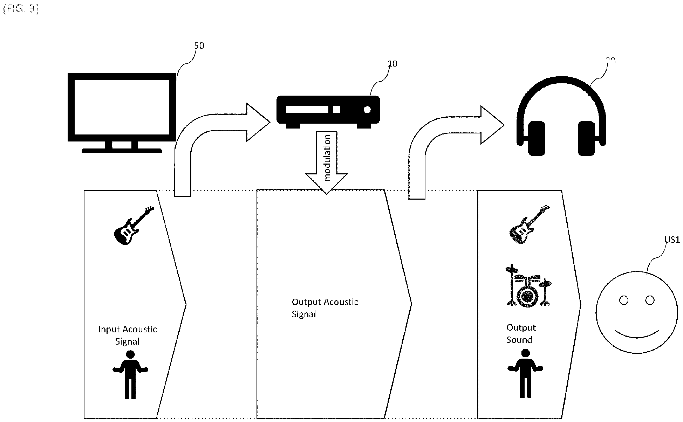

One aspect of the present embodiment will be described. FIG. 3 is an explanatory diagram of one aspect of the present embodiment.

(2-1) Outline of Embodiment

As shown in FIG. 3 , the signal processing apparatus 10 acquires an input acoustic signal from the sound source device 50 . The signal processing apparatus 10 modulates an input acoustic signal to generate an output acoustic signal. Modulation is amplitude modulation using a modulation function having a frequency corresponding to gamma waves (for example, frequencies between 35 Hz and 45 Hz). As a result, an amplitude change (volume intensity) corresponding to the frequency is added to the acoustic signal. When different modulation functions are applied to the same input acoustic signal, the amplitude waveforms of the output acoustic signals are different. Examples of amplitude waveforms will be described later.

The signal processing apparatus 10 sends the output acoustic signal to the sound output device 30 . The sound output device 30 generates an output sound according to the output acoustic signal.

A user US1 (an example of a "listener") listens to the output sound emitted from the sound output device 30 . The user US1 is, for example, a patient with dementia, a person with pre-dementia, or a healthy person who expects prevention of dementia. As mentioned above, the output acoustic signal is based on an output acoustic signal that has been modulated using a modulation function with a periodicity between 35 Hz and 45 Hz. Therefore, when the user US1 listens to the sound emitted from the sound output device 30, gamma waves are induced in the brain of the user US1. As a result, an effect of improving the cognitive function of the user US1 (for example, treating or preventing dementia) can be expected.

(2-2) First example of amplitude waveform

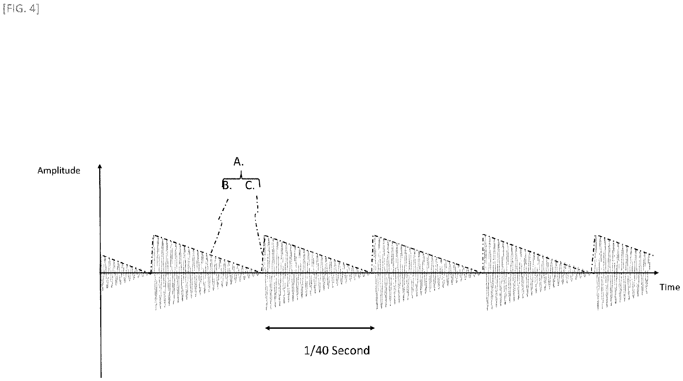

FIG. 4 is a diagram showing a first example of an amplitude waveform of an output acoustic signal. Let $A(t)$ be the modulation function used to modulate the input acoustic signal, $X(t)$ be the function representing the waveform of the input acoustic signal before modulation, and $Y(t)$ be the function representing the waveform of the output acoustic signal after modulation,

$$Y(t)=A(t)\times X(t)$$

holds.

In a first example, the modulation function has an inverse-sawtooth waveform at 40 Hz. The input acoustic signal is an acoustic signal representing a homogeneous sound with a constant frequency higher than 40 Hz and a constant sound pressure. As a result, the envelope of the amplitude waveform of the output acoustic signal has a shape along the inverse-sawtooth wave. Specifically, as shown in FIG. 4, the amplitude waveform of the output acoustic signal has an amplitude change corresponding to the frequency of the gamma wave, and the rising portion C and the falling portion B of the envelope A of the amplitude waveform are asymmetric (that is, the rising time length and the falling time length are different).

The rise of the envelope A of the amplitude waveform of the output acoustic signal in the first example is steeper than the fall. In other words, the time required for rising is shorter than the time required for falling. The amplitude value of the envelope A sharply rises to the maximum value of amplitude and then gradually falls with the lapse of time. That is, the envelope A has an inverse-sawtooth wave shape.

(2-3) Second example of amplitude waveform

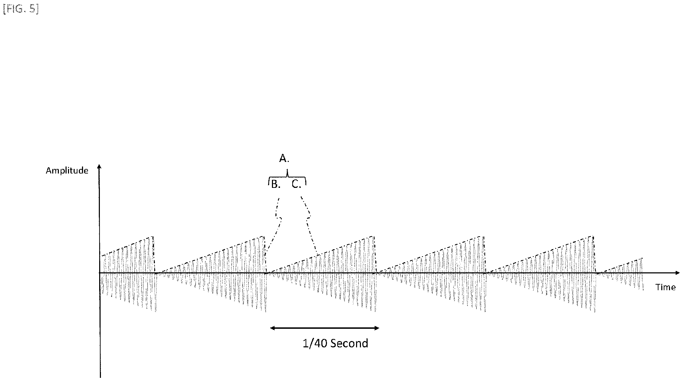

A second example of the amplitude waveform of the output acoustic signal will be described. FIG. 5 is a diagram showing a second example of the amplitude waveform of the output acoustic signal.

In a second example, the modulation function has a sawtooth waveform at 40 Hz. The input acoustic signal is an acoustic signal representing a homogeneous sound with a constant frequency higher than 40 Hz and a constant sound pressure. As a result, the envelope of the amplitude waveform of the output acoustic signal has a shape along the sawtooth wave.

Specifically, as shown in FIG. 5, the fall of the envelope A of the amplitude waveform of the output acoustic signal in the second example is sharper than the rise. In other words, the time required for falling is shorter than the time required for rising. The amplitude value of the envelope A gradually rises over time to the maximum value of the amplitude, and then sharply falls. That is, the envelope A has a sawtooth waveform.

(2-4) Third example of amplitude waveform

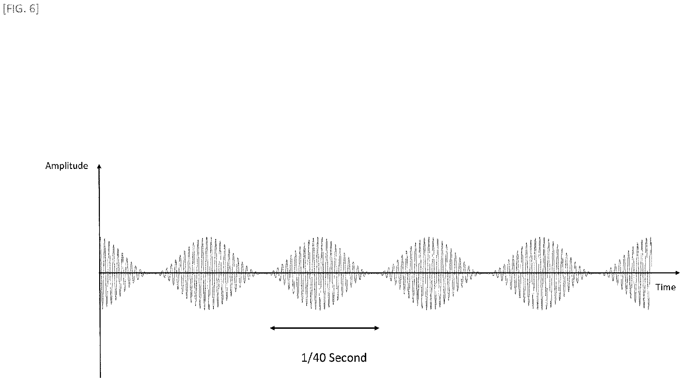

A third example of the amplitude waveform of the output acoustic signal will be described. FIG. 6 is a diagram showing a third example of the amplitude waveform of the output acoustic signal. In a third example, the modulation function has a sinusoidal waveform at 40 Hz. The input acoustic signal is an acoustic signal representing a homogeneous sound with a constant frequency higher than 40 Hz and a constant sound pressure. As a result, the envelope of the amplitude waveform of the output acoustic signal has a shape along the sinusoidal wave.

Specifically, as shown in FIG. 6, both the rise and fall of the envelope A of the amplitude waveform of the output acoustic signal in the third example are smooth. That is, the envelope A is sinusoidal.

In the first to third examples above, the modulation function has a periodicity of 40 Hz, but the frequency of the modulation function is not limited to this, and may be, for example, a frequency between 35 Hz and 45 Hz. In addition, in the above first to third examples, the absolute value of the amplitude value of the envelope A is periodically set to 0, but this is not limiting, and a modulation function may be used such that the minimum absolute value of the amplitude value of the envelope A is greater than 0 (e.g., half or quarter the maximum absolute value).

Although the sound pressure and frequency of the input acoustic signal are constant in the examples shown in FIGS. 4 to 6, the sound pressure and frequency of the input acoustic signal may vary. For example, the input acoustic signal may be a signal representing music, speech, environmental sounds, electronic sounds, or noise. In this case, the envelope of the amplitude waveform of the output acoustic signal is strictly different in shape from the waveform representing the modulation function, but the envelope has a rough shape similar to that of the waveform representing the modulation function (for example, an inverse-sawtooth wave, a sawtooth wave, or sinusoidal), and can provide the listener with the same auditory stimulus as when the sound pressure and frequency of the input acoustic signal are constant.

(2-5) Experimental results

(2-5-1) Outline of experiment

Experiments conducted to verify the effects of the technique of the present disclosure will be described.

In this experiment, 18 male and 8 female subjects were made to listen to an output sound based on an acoustic signal modulated using a modulation function of 40 Hz, and the psychological reaction they felt during the sound, as well as the degree of gamma wave induction in their brains, were also evaluated. For comparison, the degree of psychological reactoin and induction of gamma waves was also evaluated when the subjects were made to listen to an output sound based on an acoustic signal having a 40-Hz pulse-like waveform. The psychological reaction was evaluated based on the subjects' subjective responses to a questionnaire (7-grade scale). The degree of gamma wave induction was measured by multiple electrodes attached to the subject's head. Headphones worn on the subject's head were used as the sound output device 30 that generates an output sound in accordance with the modulated acoustic signal.

In this experiment, the following experiments A to C were performed in order.

- Experiment A: An experiment in which subjects answered their discomfort when hearing multiple types of output sounds corresponding to different modulation functions and input acoustic signals.
- Experiment B: An experiment to measure the electroencephalogram of a subject when listening to multiple types of output sounds corresponding to different modulation functions and input acoustic signals.
- Experiment C: An experiment to measure the electroencephalogram of the subject when listening to multiple types of output sounds corresponding to different modulation functions and input acoustic signals (the length of the sound is different from Experiment B).

In Experiment A, a 7-point scale evaluation questionnaire was conducted to determine how well the following items apply.

- I feel uncomfortable

- I feel irritated

- Sound is unnatural

- Sound is difficult to hear

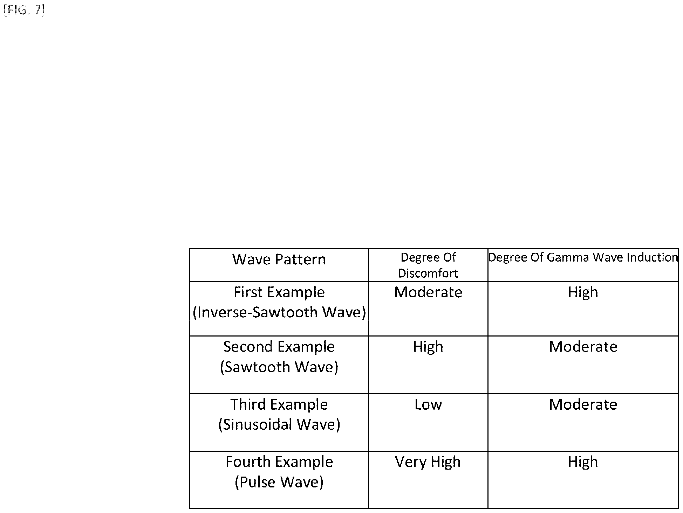

In this experiment, an acoustic signal modulated using modulation functions corresponding to the first example of the amplitude waveform, the second example of the amplitude waveform, and the third example of the amplitude waveform, respectively, and a fourth example of acoustic signal with a pulse-like waveform were tested. The evaluation was carried out using four acoustic signals. FIG. 7 is a diagram showing the results of the experiment.

As shown in FIG. 7, gamma wave induction was confirmed in all modulated waveform patterns. Therefore, it can be expected that gamma waves are induced in the brain of the user US1 when the user US1 listens to the sound emitted from the sound output device 30 in the present embodiment. By inducing gamma waves in the brain of the user US1, an effect of improving the cognitive function of the user US1 (for example, treatment or prevention of dementia) can be expected. Further, it was confirmed that the waveform patterns of the first to third examples caused less discomfort than the waveform pattern of the fourth example. From this, when using the acoustic signal modulated by the modulation function disclosed in this embodiment, the discomfort given to the listener when listening to the sound can be expected to be suppressed more than when using the acoustic signal composed of a simple pulse wave.

Moreover, as shown in FIG. 7, it was confirmed that the degree of induction of gamma waves was highest in the waveform pattern of the first example compared to the second and third examples. On the other hand, it was confirmed that the degree of discomfort was lowest in Example 3 and highest in Example 2. For this reason, when the envelope of the amplitude waveform of the output acoustic signal output from the signal processing apparatus 10 to the sound output device 30 in the present embodiment becomes an inverse-sawtooth wave, it can be expected to achieve high cognitive function improvement effects while suppressing discomfort given to the listener. Further, in this embodiment, when the envelope of the amplitude waveform of the output acoustic signal output from the signal processing apparatus 10 to the sound output device 30 is sinusoidal, it can be expected that the discomfort given to the listener will be further suppressed.

(2-5-2) Experiment details

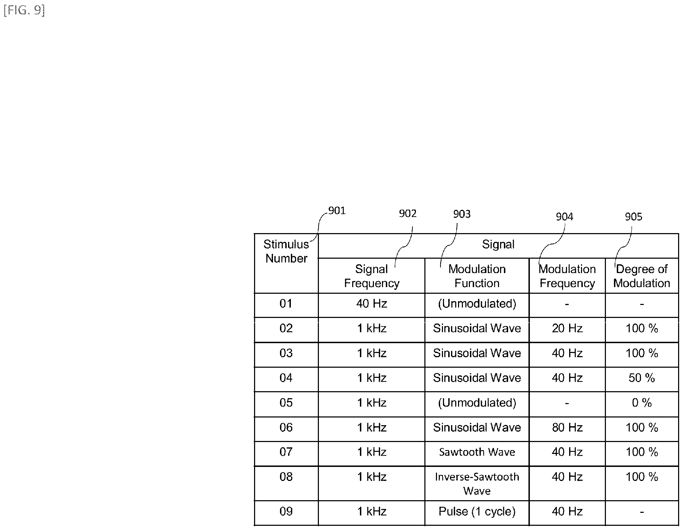

The above experiments for verifying the effect of the technique of the present disclosure will be described in further detail. In the following, the above-mentioned Experiments A and B will be mainly described, and Experiment C will be omitted. In this experiment, attention is paid to electroencephalograms having a frequency of 40 Hz as gamma waves to be induced. FIG. 9 shows a list of sound stimuli (output sounds) used in this experiment. Column 901 shows the identification number of the sound stimulus (hereinafter referred to as "stimulus number"), column 902 shows the frequency of the acoustic signal (sinusoidal wave) before modulation, column 903 shows whether it is modulated or not and the modulation function used for modulation, column 904 shows the frequency of the modulation function and column 905 shows the degree of modulation.

Nine types of stimuli were prepared as stimulus groups using sinusoidal waves (stimulus numbers "01" to "09"). First, a sinusoidal wave of 40 Hz was created for comparison (stimulus number "01"). The stimulus is pure sinusoidal and unmodulated. A continuous sinusoidal wave of 1 kHz was then modulated. Modulation was performed by multiplying a sinusoidal wave of 1 kHz with the following envelopes. For the envelopes, a sawtooth wave and an inverse-sawtooth wave were used in addition to a normal sinusoidal wave (so-called AM modulation). Stimulus numbers "02" to "06" are sinusoidally modulated sound stimuli. The envelope of sinusoidal modulation is represented by Equation (1).

$$env_{sinusoidal}(t) = \left(1 + m \cdot \sin(2\pi f_m t)\right), \qquad (1)$$

Here, m is the degree of modulation, and 0.00, 0.50 and 1.00 are used. fm is a modulation frequency, and 20 Hz, 40 Hz and 80 Hz are used. t is time. A sinusoidally modulated sound stimulus corresponds to the third example of the amplitude waveform described above. Next, stimulus numbers "07" and "08" are a sawtooth-wave-modulated sound stimulus and an inverse-sawtooth-wave-modulated sound stimulus, respectively. Envelopes of sawtooth wave modulation and inverse-sawtooth wave modulation are represented by equations (2) and (3), respectively.

$$env_{sawtooth}(t) = \left(1 + m \cdot \text{sawtooth}(2\pi f_m t)\right) \qquad (2)$$

$$env_{inversesawtooth}(t) = \left(1 - m \cdot \text{sawtooth}(2\pi f_m t)\right), \qquad (3)$$

The modulation degree m was set to 1.00, and the modulation frequency fm was set to 40 Hz. A sawtooth-wave-modulated sound stimulus and an inverse-sawtooth-wave-modulated sound stimulus correspond to the second and first examples of amplitude waveforms described above, respectively. The sawtooth function used here is a discontinuous function that repeatedly increases linearly from -1 to 1 and then instantly returns to -1. The stimuli used in the experiments were adjusted to have equal equivalent noise levels (Laeq) after modulation. For example, the 40-Hz sinusoidal wave of stimulus number "01" has a sound pressure level 34.6 dB higher than that of 1 kHz when the equivalent noise level is aligned, but this aligns the auditory loudness.

In addition to these, the stimulus used in the study of Literature 1 (a pulse train consisting of one wavelength of 1 kHz sinusoidal wave with a taper of 0.3 ms before and after, repeated at a period of 40 Hz) was used as a comparison target (stimulus number "09"). This pulse wave-like sound stimulation corresponds to the fourth example of the amplitude waveform described above. This stimulus also had the same equivalent noise level as the stimulus numbers "01" to "08".

After that, in order to prevent noise at the start and end of reproduction, taper processing was applied for 0.5 seconds each before and after stimulation. By performing the taper processing at the end in this manner, the equivalent noise level in the steady section is strictly maintained. The duration of stimulation was 10 seconds for psychological experiments and 30 seconds for electroencephalogram measurements.

All of these stimuli were presented to the experimental participants in mono (diotic) through headphones at Laeq=60 dB. That is, the sound pressure level was adjusted to 60 dB for a sinusoidal wave of 1 kHz without modulation. Sound pressure calibration was performed with a dummy head. The participants of the experiment were 26 persons (22.7±2.1 years old, 18 males and 8 females) who were native speakers of Japanese and had normal hearing.

Prior to the electroencephalogram measurement experiment, a psychological evaluation experiment for each stimulus (corresponding to experiment A described above) was conducted. The stimulus length including the taper is 10 seconds. Since all participants heard these stimuli for the first time in this psychological experiment, it is thought that the effects of habituation to each stimulus were minimal.

The experiment was conducted in the same quiet magnetically shielded room as the electroencephalogram measurement experiment, with headphone presentation. An LCD display was installed in front of the experiment participants, and a GUI was prepared for psychological evaluation. All responses were made by mouse operation. As a question item, the degree of discomfort and irritation when listening to each sound stimulus were evaluated on a 7-point scale. Playback was limited to one time, and the UI was designed so that no response could be given until the 10-second stimulus had finished playing. The next stimulus was set to play automatically when the response was completed. It was also designed to automatically prompt them to take a break in the middle of the experiment.

The participants were asked to imagine that the sound they were listening to in this experiment was being played as the sound of a TV program, video distribution service, radio, etc., and that they were listening to it in their living room. Judge how unnatural the sound is, or how difficult it is to hear." We also explained that the meaning of the words played was irrelevant to the experiment. Prior to the experiment, a practice task was performed using four stimuli.

In the experiment, the 7-level answers for each subject were regarded as a numerical scale of 1 to 7, and arithmetically averaged, and these values were averaged among the subjects.

After the psychological experiment, electroencephalogram measurement (corresponding to Experiment B above) was performed. Measurements were performed in a quiet, magnetically shielded room. The length of the stimulus used, including the taper, was 30 seconds. During the experiment, stimuli with the same treatment were presented twice. The interstimulus interval was 5 seconds, and the order of presentation was random. Experimental participants were instructed to move as little as possible and blink as little as possible during the presentation of the stimulus. In addition, a silent short animation video was played on an LCD monitor, and the level of consciousness was controlled to be constant and the level of attention to be stably lowered. Participants in the experiment were asked to select a video from among those prepared in advance. In addition to the A1 and A2 reference electrodes, the experimental participants were provided with active electrodes at the positions of Fp1, Fp2, F3, F4, T3, T4, T5, T6, Cz and Pz channels of the 10-20 method, respectively.

The measured EEG waveforms were analyzed after the experiment. First, of the 30-second stimulus presentation interval, the 1 second regional tapers at the beginning and end were excluded from the analysis target. After that, 55 sections of 1 second were cut out while shifting by 0.5 seconds. Since the same processing is performed twice, the analysis target is 110 sections. FFT was performed by applying a Hann window to each of these 110 waveforms. Since the Hann window is moved half by half and the Hann window is applied, the data at all times are treated equally as a result. For the FFT results, the ratio of the power of the 40 Hz component to the sum of the power of the 14 Hz to 100 Hz components is calculated, averaged over 110 intervals, and one scalar value for each electrode of each experiment participant (40 Hz EEG power spectrum ratio.) was obtained. First, for each channel, the average value and standard deviation between subjects were calculated for each response to each stimulus. Based on this data, we investigated which side of the brain the brain wave evoked was dominant, and selected one representative electrode that appeared to be largely unaffected by electrical noise in that area. A hypothesis test was performed on this electrode value. For each stimulus group, differences were confirmed by analysis of variance (ANOVA), and differences were tested by multiple comparison by Tukey's method.

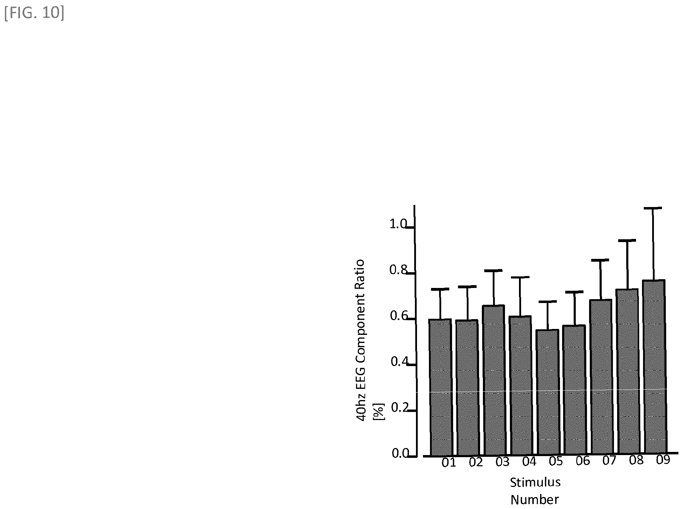

FIG. 10 is a diagram showing experimental results of electroencephalogram evoked by sound stimulation. Specifically, FIG. 10 shows the power ratio of the 40 Hz component of the electroencephalogram evoked by each stimulus in the T6 channel. Values and error bars in the graph are the mean and standard deviation for all experimental participants. ANOVA confirmed a significant difference in stimulation ($p<0.01$).

First, the stimulus using a sinusoidal wave will be described. Using the non-modulated 1 kHz sinusoidal wave (stimulus number "05") as a reference, there was no difference in the 40 Hz component of the electroencephalogram between the 40 Hz sinusoidal wave (stimulus number "01") ($p=0.94$). The equivalent noise levels of these stimuli are aligned, and the loudness is also approximately aligned. In other words, it suggests that simply presenting a low-frequency sound of 40 Hz cannot induce an electroencephalogram of a 40-Hz component. It was also shown that the 40 Hz component signal applied to the headphone was not detected as electrical noise by the electroencephalogram electrodes. This is because the 40 Hz component is included most in stimulus number "01". It was also found that the 20 Hz sinusoidal wave modulation (stimulus number "02") and the 80 Hz sinusoidal wave modulation (stimulus number "06") did not induce the 40 Hz component. In addition, with 40 Hz sinusoidal modulation (stimulus numbers "03", "04" and "05"), a tendency is observed that 40 Hz components are evoked according to the degree of modulation.

In contrast, the sawtooth wave modulation (stimulus number "07") and the inverse-sawtooth wave modulation (stimulus number "08") were both significantly different from the unmodulated 1 kHz sinusoidal wave (stimulus number "05"). Also, no significant difference was found between these two stimuli. Therefore, it is shown that even a sound of 1 kHz, not a low frequency sound of 40 Hz, can induce a brain wave component of 40 Hz in the brain by setting the amplitude envelope curve of the modulation function to 40 Hz. In addition, the pulsed stimulus (stimulus number '09') was also significantly different from the unmodulated 1 kHz sinusoidal wave (stimulus number '05'). Also, there was no significant difference between the pulsatile stimulus (stimulus number "09") and the sawtooth wave modulation and inverse-sawtooth wave modulation (stimulus numbers "07" and "08"). These data show that similar electroencephalographic effects can be obtained not only with pulsed sounds but also with amplitude-modulated sinusoidal waves (sounds that maintain the pitch of the original 1 kHz sinusoidal wave). is shown.

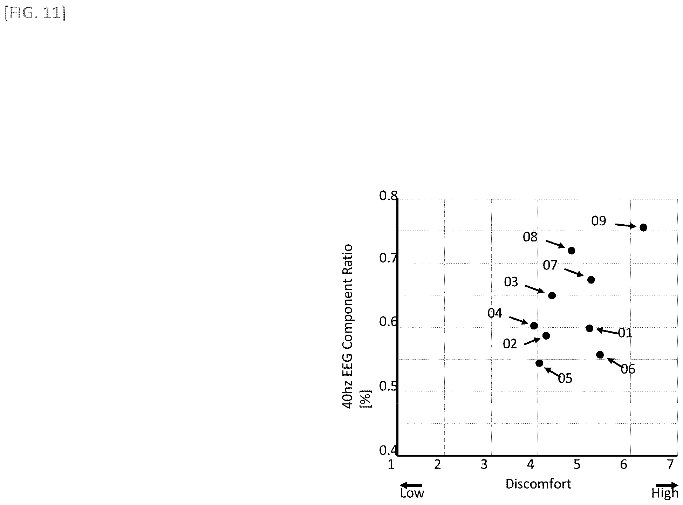

Finally, the relationship between the values of the psychological experiment results and the electroencephalogram measurement results is examined. FIG. 11 is a diagram showing the correlation between the results of psychological experiments and electroencephalogram measurements. Specifically, FIG. 11 shows the relationship between the degree of discomfort and the 40 Hz electroencephalogram component ratio.

A positive correlation is observed between the degree of discomfort and the 40 Hz electroencephalogram ratio ($r=0.56$). From this, it can be seen that there is a general tendency, at least in the stimulation group used this time, that the higher the degree of discomfort, the greater the evoked 40-Hz electroencephalogram. For example, it can be seen that the pulsating stimulus of stimulus number "09" causes a very high degree of discomfort, but at the same time, 40 Hz electroencephalograms are also evoked to the greatest extent. However, since the correlation is not so strong, there are stimuli that deviate greatly from the regression line even if the regression line is drawn. For example, stimulus number "08", which is a stimulus obtained by modulating a sinusoidal wave with an inverse-sawtooth wave, is located above the regression line, and it shows a great decrease in discomfort than that of stimulus number "09", but the degree of decrease in the 40 Hz electroencephalogram ratio is smaller than the other stimuli. Conversely, with stimulation number "06", which is a sinusoidal wave modulated by 80 Hz sinusoidal waves, the decrease in discomfort is small, but the decrease in 40 Hz electroencephalograms is significant. In addition, stimulus number "07", which is a sinusoidal wave modulated by sawtooth wave modulation, has lower discomfort level and 40 Hz electroencephalogram ratio than stimulus number "09", which is pulse-type stimulation, and a slightly higher discomfort level and smaller 40 Hz electroencephalogram ratio than inverse-sawtooth wave modulation stimulus number "08". is smaller. Stimulus number "03", which is a stimulus obtained by modulating a 1 kHz sinusoidal wave with a 40 Hz sinusoidal wave at a modulation degree of 100%, has a lower discomfort level and 40 Hz electroencephalogram ratio than stimulus number "09", which is a pulsed stimulus, and a slightly smaller discomfort level and 40Hz electroencephalogram ratio than inverse-sawtooth wave modulation stimulus number "08".

(3) Acoustic signal processing

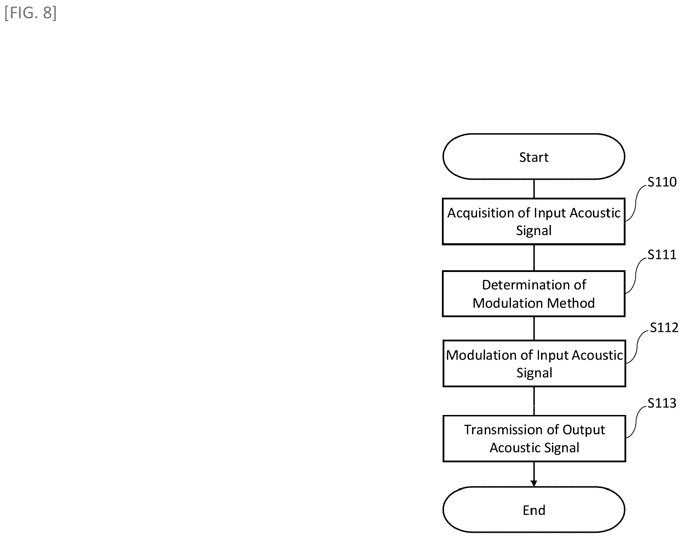

Acoustic signal processing according to this embodiment will be described. FIG. 8 is a diagram showing the overall flow of acoustic signal processing by the signal processing apparatus 10 of this embodiment. The processing in FIG. 8 is implemented by the processor 12 of the signal processing apparatus 10 reading and executing the program stored in the storage device 11 . At least part of the processing in FIG. 8 may be realized by one or more dedicated circuits.
The acoustic signal processing in FIG. 8 is started when any of the following start conditions is satisfied.

- The acoustic signal processing of FIG. 8 was called by another process or an instruction from the outside.
- The user performed an operation to call the acoustic signal processing in FIG. 8.
- The signal processing apparatus 10 has entered a predetermined state (for example, the power has been turned on).
- The specified date and time has arrived.
- A predetermined time has passed since a predetermined event (for example, activation of the signal processing apparatus 10 or previous execution of the acoustic signal processing in FIG. 8).

As shown in FIG. 8, the signal processing apparatus 10 executes acquisition of an input acoustic signal (S110).
Specifically, the signal processing apparatus 10 receives an input acoustic signal sent from the sound source device 50 .
In step S110, the signal processing apparatus 10 may further perform A/D conversion of the input acoustic signal.
The input acoustic signal corresponds, for example, to at least one of the following:

- Musical content (e.g., singing, playing, or a combination thereof (i.e., a piece of music). It may include audio content that accompanies the video content. );
- Voice content (for example, reading, narration, announcement, broadcast play, solo performance, conversation, monologue, or a combination thereof, etc. It may include audio content accompanying video content); and
- Other acoustic content (e.g., electronic, ambient, or mechanical sounds).

However, singing or voice content is not limited to sounds produced by human vocal organs, but may include sounds generated by speech synthesis technology.
After step S110, the signal processing apparatus 10 executes determination of the modulation method (S111).
Specifically, the signal processing apparatus 10 determines the modulation method used to generate the output acoustic signal from the input acoustic signal acquired in step S110. The modulation method determined here includes, for example, at least one of a modulation function used for modulation processing and a degree of modulation corresponding to the degree of amplitude change due to modulation. As an example, the signal processing apparatus 10 selects which one of the three types of modulation functions described with reference to FIGS. 4 to 6 is to be used. Which modulation function to select may be determined based on an input operation by the user or other person, or an instruction from the outside, or may be determined by an algorithm.
In this embodiment, the other person is, for example, at least one of the following:

User's family, friends, or acquaintances;
Medical personnel (for example, the user's doctor);
Creator or provider of content corresponding to the input acoustic signal;
The provider of the signal processing apparatus 10; and
Administrators of facilities used by users.

The signal processing apparatus 10 may determines the modulation method based on, for example, at least one of the characteristics of the input acoustic signal (balance between voice and music, volume change, type of music, timbre, or other characteristics) and user attribute information (age, gender, hearing ability, cognitive function level, user identification information and other attribute information). Thereby, the signal processing apparatus 10 can determine the modulation method so that the effect of improving the cognitive function by modulation becomes higher, or determine the modulation method so as to make the user less uncomfortable. Further, for example, the signal processing apparatus 10 may determine the modulation method according to a timer. By periodically changing the modulation method according to the timer, it is possible to prevent the user from becoming accustomed to listening to the modulated sound, and to efficiently stimulate the user's brain. Further, the signal processing apparatus 10 may determine the volume of the output acoustic signal according to various conditions, similar to determining the modulation method.

In step S111, the signal processing apparatus 10 may decide not to perform modulation (that is, set the degree of modulation to 0) as one of the options for the modulation method. Further, the signal processing apparatus 10 may determine the modulation method so that the modulation is performed when a predetermined time has elapsed after the modulation method is determined so as not to perform the modulation. Furthermore, the signal processing apparatus 10 may determine the modulation method so that the degree of modulation gradually increases when changing from a state in which no modulation is performed to a state in which modulation is performed.

After step S111, the signal processing apparatus 10 executes modulation of the input acoustic signal (S112) to generate an output acoustic signal.

Specifically, the signal processing apparatus 10 performs modulation processing according to the modulation method determined in Sill on the input acoustic signal acquired in S110. As an example, the signal processing apparatus 10 amplitude-modulates the input acoustic signal using a modulation function having a frequency corresponding to a gamma wave (for example, a frequency between 35 Hz and 45 Hz). As a result, an amplitude change corresponding to the frequency is added to the input acoustic signal.

In step S112, the signal processing apparatus 10 may further perform at least one of amplification, volume control, and D/A conversion of the output acoustic signal.

After step S112, the signal processing apparatus 10 executes transmission of an output acoustic signal (S113).

Specifically, the signal processing apparatus 10 sends the output acoustic signal generated in step S112 to the sound output device 30 . The sound output device 30 generates sound according to the output acoustic signal.

The signal processing apparatus 10 ends the acoustic signal processing in FIG. 8 at step S113.

Note that the signal processing apparatus 10 may collectively perform the processing in FIG. 8 for an input acoustic signal having a certain reproduction period (for example, music content of one piece of music), or may repeat the processing in FIG.8 for each predetermined reproduction interval of the input acoustic signal (for example, every 100 ms). Alternatively, the signal processing apparatus 10 may continuously perform modulation processing on an input acoustic signal, such as modulation by analog signal processing, and output a modulated acoustic signal. The processing shown in FIG. 8 may be terminated according to a specific termination condition (for example, a certain period of time has passed, a user operation has been performed, or the output history of modulated sound has reached a predetermined state).

The order of processing by the signal processing apparatus 10 is not limited to the example shown in FIG. 8, and for example, the determination of the modulation method (S111) may be performed before the acquisition of the input acoustic signal (S110).

(4) Summary

As described above, the signal processing apparatus 10 of the present embodiment amplitude-modulates an input acoustic signal to generate an output acoustic signal having an amplitude change corresponding to the gamma wave frequency. In the output acoustic signal, the rise and fall of the envelope of the amplitude waveform are asymmetrical. The signal processing apparatus 10 outputs the generated output acoustic signal to the sound output device 30 . As a result, the amplitude of the acoustic signal can be increased or decreased in a predetermined cycle while suppressing discomfort given to the listener. Then, the sound output device 30 causes the user to listen to the sound corresponding to the output acoustic signal, thereby inducing gamma waves in the user's brain due to fluctuations in the amplitude of the output acoustic signal. As a result, the effect of improving the user's cognitive function (for example, treating or preventing dementia) can be expected.

The output acoustic signal may have amplitude variations corresponding to frequencies between 35 Hz and 45 Hz. As a result, when the user hears the sound corresponding to the output acoustic signal, it can be expected that gamma waves will be induced in the user's brain.

The input acoustic signal may be an acoustic signal corresponding to music content. As a result, the motivation of the user to listen to the sound corresponding to the output acoustic signal can be improved.

In the experiment, it was confirmed that gamma waves were induced in any of the three patterns of waveforms.

Thus, according to the input acoustic signal with amplitude waveforms in the first, second, and third examples, it can be expected to improve the user's cognitive function (for example, treatment or prevention of dementia).

(5) Modification

The storage device 11 may be connected to the signal processing apparatus 10 via the network NW. The display 21 may be built in the signal processing apparatus 10.

In the above description, an example in which the signal processing apparatus 10 modulates the input acoustic signal was shown. However, the signal processing apparatus 10 may extract a part of the acoustic signal from the input acoustic signal, modulate only the extracted acoustic signal, and then generate the output acoustic signal.

The above explanation shows an example in which the signal processing apparatus 10 sends the output acoustic signal generated by modulating the input acoustic signal to the sound output device 30. However, the signal processing apparatus 10 may generate an output acoustic signal by synthesizing another acoustic signal to a modulated input acoustic signal obtained by modulating the input acoustic signal, and send the generated output acoustic signal to the sound output device 30.

Further, the signal processing apparatus 10 may send the modulated input acoustic signal and another acoustic signal to the sound output device 30 at the same time without synthesizing them.

The above explanation shows an example in which the envelope of the amplitude waveform is an inverse-sawtooth wave or a sawtooth wave in the output acoustic signal generated by the signal processing apparatus 10 modulating the input acoustic signal, and the rise and fall of the envelope are asymmetrical. However, the output acoustic signal generated by the signal processing apparatus 10 is not limited to these, and may have other amplitude waveforms in which the rise and fall of the envelope of the amplitude waveform are asymmetrical.

For example, in the rising portion of the envelope, the slope of the tangent to the envelope may gradually decrease, or the slope of the tangent to the envelope may gradually increase. Further, for example, in the falling portion of the envelope, the slope of the tangent to the envelope may gradually decrease, or the slope of the tangent to the envelope may gradually increase.

In the above description, an example in which the modulation function has a frequency between 35 Hz and 45 Hz has been mainly described. However, the modulation function used by the signal processing apparatus 10 is not limited to this, and any modulation function that affects the induction of gamma waves in the brain of the listener may be used. For example, the modulation function may have frequencies between 25 Hz and 140 Hz. For example, the frequency of the modulating function may change over time, and the modulating function may have a frequency below 35 Hz or a frequency above 45 Hz in part.

In the above description, the case where the output acoustic signal generated by the signal processing apparatus 10 is output to the sound output device 30 that emits a sound corresponding to the output acoustic signal for the user to hear has been described. However, the output destination of the output acoustic signal by the signal processing apparatus 10 is not limited to this. For example, the signal processing apparatus 10 may output the output acoustic signal to an external storage device or information processing apparatus via a communication network or by broadcasting. At this time, the signal processing apparatus 10 may output the input acoustic signal that has not been modulated together with the output acoustic signal generated by the modulation processing to an external device. As a result, the external device can arbitrarily select and reproduce one of the unmodulated acoustic signal and the modulated acoustic signal.

Further, the signal processing apparatus 10 may output information indicating the content of modulation processing to an external device together with the output acoustic signal. Information indicating the content of modulation processing includes, for example, any of the following:

- Information indicating the modulation function;
- Information indicating the degree of modulation; and
- Information indicating volume.

Thereby, the external device can change the reproduction method of the acoustic signal according to the detail of the modulation processing.

Further, when the signal processing apparatus 10 acquires additional information (for example, an ID3 tag in an MP3 file) together with the input acoustic signal, the signal processing apparatus 10 may change the additional information and output it to the external device together with the output acoustic signal.

In the above-described embodiment, the case where the acoustic system 1 including the signal processing apparatus 10 is used as a cognitive function improvement system for improving cognitive function (for example, treatment or prevention of dementia) has been mainly described. However, the application of the signal processing apparatus 10 is not limited to this. Literature 1 discloses that when 40-Hz sound stimulation induces gamma waves in the brain, amyloid B is reduced and cognitive function is improved. That is, by making the user hear the sound corresponding to the output acoustic signal output by the signal processing apparatus 10, the amount of amyloid B in the brain of the user is reduced and the deposition is suppressed. It is expected to be useful for the prevention or treatment of various diseases caused by increased amyloid B or depositon of amyloid B. Diseases caused by deposition of amyloid B include, for example, cerebral amyloid angiopathy (CAA). CAA is a disease in which amyloid B protein deposits on the walls of small blood vessels in the brain, making the walls of blood vessels fragile and causing cerebral hemorrhage and the like. As with dementia, there is no therapeutic drug for CAA itself, so the technology described in the above embodiments can be an innovative therapeutic method. That is, the acoustic system 1 comprising the signal processing apparatus 10 and the sound output device 30 for allowing the user to hear a sound corresponding to the output acoustic signal output by the signal processing apparatus 10 can also be used as a medical system for treatment or prevention of cerebral amyloid angiopathy.

Although the embodiments of the present invention are described in detail above, the scope of the present invention is not limited to the above embodiments. Further, various improvements and modifications can be made to the above embodiments without departing from the spirit of the present invention. In addition, the above embodiments and modifications can be combined.

[Reference Signs List]

1: Acoustic system
10: Signal processing apparatus
11: Storage device
12: Processor
13: Input/output interface
14: Communication interface
21: Display
30: Sound output device
50: Sound source device

**Claims**

1.  A signal processing apparatus, comprising:

    means for receiving an input acoustic signal;
    means for amplitude-modulating the received input acoustic signal to generate an output acoustic signal having an amplitude change corresponding to a frequency of a gamma wave and having an asymmetric rising and falling edge of the envelope of the amplitude waveform; and
    means for outputting the generated output acoustic signal.

2.  The signal processing apparatus according to claim 1, wherein
    the rising edge of the envelope of the amplitude waveform in the output acoustic signal is steeper than the falling edge of the envelope.

3.  The signal processing apparatus according to claim 2, wherein
    the envelope of the amplitude waveform of the output acoustic signal is a reverse sawtooth waveform.

4.  The signal processing apparatus according to claim 1, wherein
    the fall of the envelope of the amplitude waveform in the output acoustic signal is steeper than the rise.

5.  The signal processing apparatus according to claim 4, wherein
    the envelope of the amplitude waveform of the output acoustic signal is sawtooth.

6.  A signal processing apparatus, comprising:

    means for receiving an input acoustic signal;
    means for amplitude-modulating the received input acoustic signal to generate an output acoustic signal having a change in amplitude corresponding to the frequency of the gamma wave and having a sinusoidal envelope of the amplitude waveform; and
means for outputting the generated output acoustic signal.

7. The signal processing apparatus according to any one of claims 1 to 6, wherein
the output acoustic signal has an amplitude change corresponding to a frequency of 35 Hz or more and 45 Hz or less.

8. The signal processing apparatus according to any one of claims 1 to 6, wherein
the input audio signal is an audio signal corresponding to music content.

9. The signal processing apparatus according to claim 1, comprising:
means for causing a user to hear a sound corresponding to the output acoustic signal output by the signal processing apparatus.

10. A signal processing method, comprising:

receiving an input acoustic signal;
amplitude-modulating the received input acoustic signal to generate an output acoustic signal having an amplitude change corresponding to the frequency of the gamma wave and having an asymmetric rising and falling edge of the envelope of the amplitude waveform; and
outputting the generated output acoustic signal.

11. A program for causing a computer to execute the signal processing method according to claim 10.

[FIG. 1]

1

```
┌─────────────────┐
│  Sound Source   │
│     Device      │
│       50        │
└─────────────────┘
         │
         │
┌─────────────────┐
│Signal Processing│
│   Apparatus     │
│       10        │
└─────────────────┘
         │
         │
┌─────────────────┐
│  Sound Output   │
│     Device      │
│       30        │
└─────────────────┘
```

[FIG. 2]

Display
21

10

Storage device
11

Input And Output
Interface
13

Processor
12

Communication
Interface
14

[FIG. 3]

Input Acoustic Signal

Output Acoustic Signal

modulation

Output Sound

US1

[FIG. 4]

[FIG. 5]

[FIG. 6]

Amplitude

Time

1/40 Second

[FIG. 7]

| Wave Pattern | Degree Of Discomfort | Degree Of Gamma Wave Induction |
|---|---|---|
| First Example (Inverse-Sawtooth Wave) | Moderate | High |
| Second Example (Sawtooth Wave) | High | Moderate |
| Third Example (Sinusoidal Wave) | Low | Moderate |
| Fourth Example (Pulse Wave) | Very High | High |

[FIG. 8]

```
         ┌─────────────────┐
         │      Start       │
         └─────────────────┘
                  │                         S110
         ┌─────────────────┐
         │ Acquisition of Input Acoustic │
         │         Signal         │
         └─────────────────┘
                  │                         S111
         ┌─────────────────┐
         │  Determination of    │
         │  Modulation Method   │
         └─────────────────┘
                  │                         S112
         ┌─────────────────┐
         │ Modulation of Input Acoustic │
         │         Signal         │
         └─────────────────┘
                  │                         S113
         ┌─────────────────┐
         │ Transmission of Output │
         │    Acoustic Signal     │
         └─────────────────┘
                  │
         ┌─────────────────┐
         │       End        │
         └─────────────────┘
```

[FIG. 9]

| Stimulus Number | Signal | | | |
|---|---|---|---|---|
| | Signal Frequency | Modulation Function | Modulation Frequency | Degree of Modulation |
| 01 | 40 Hz | (Unmodulated) | - | - |
| 02 | 1 kHz | Sinusoidal Wave | 20 Hz | 100 % |
| 03 | 1 kHz | Sinusoidal Wave | 40 Hz | 100 % |
| 04 | 1 kHz | Sinusoidal Wave | 40 Hz | 50 % |
| 05 | 1 kHz | (Unmodulated) | - | 0 % |
| 06 | 1 kHz | Sinusoidal Wave | 80 Hz | 100 % |
| 07 | 1 kHz | Sawtooth Wave | 40 Hz | 100 % |
| 08 | 1 kHz | Inverse-Sawtooth Wave | 40 Hz | 100 % |
| 09 | 1 kHz | Pulse (1 cycle) | 40 Hz | - |

[FIG. 10]

[FIG. 11]

<div align="center">

**INTERNATIONAL SEARCH REPORT**

</div>

| International application No. |
|---|
| **PCT/JP2022/039422** |

**A.    CLASSIFICATION OF SUBJECT MATTER**

*G10L 21/0364*(2013.01)i
FI:    G10L21/0364

According to International Patent Classification (IPC) or to both national classification and IPC

**B.    FIELDS SEARCHED**

Minimum documentation searched (classification system followed by classification symbols)

A61B5/12; A61M21/00-21/02; G10H1/00-1/46; G10K15/00-15/12; G10L21/00-21/18

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| X | 安井希子, 三浦雅展, AM音の振幅形状が"Roughness知覚を変えるのか?, 日本音響学会講演論文集, March 2011, pages 1013-1016, ISSN 1880-7658, (YASUI, Nozomiko, IURA, Masanobu. Acoustical Society of Japan.), non-official translation (Does the Amplitude Shape of AM Sounds Change Roughness Perception?) | 1-7, 10-11 |
| | chapter 2-3 | |
| A | | 8-9 |
| X | JP 57-032497 A (MATSUSHITA ELECTRIC IND. CO., LTD.) 22 February 1982 (1982-02-22) | 1-8, 10-11 |
| | page 1, left column, lines 10-13, right column, line 4 to page 2, left column, line 15 | |
| Y | | 9 |
| Y | JP 2020-014716 A (FAITH INC.) 30 January 2020 (2020-01-30) paragraphs [0002], [0045]-[0047] | 9 |
| A | JP 2011-251058 A (PANASONIC CORP.) 15 December 2011 (2011-12-15) entire text, all drawings | 1-11 |

☑ Further documents are listed in the continuation of Box C.　　　☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **05 December 2022** | **20 December 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2022/039422**

| C. | DOCUMENTS CONSIDERED TO BE RELEVANT | |
|---|---|---|
| Category* | Citation of document, with indication, where appropriate, of the relevant passages | Relevant to claim No. |
| A | JP 55-031595 Y1 (NIPPON GAKKI CO., LTD.) 28 July 1980 (1980-07-28) entire text, all drawings | 1-11 |
| A | US 4160402 A (SCHWARTZ, Louis A.) 10 July 1979 (1979-07-10) entire text, all drawings | 1-11 |

Form PCT/ISA/210 (second sheet) (January 2015)

INTERNATIONAL SEARCH REPORT
Information on patent family members

International application No.

**PCT/JP2022/039422**

| Patent document cited in search report | | | Publication date (day/month/year) | Patent family member(s) | Publication date (day/month/year) |
|---|---|---|---|---|---|
| JP | 57-032497 | A | 22 February 1982 | (Family: none) | |
| JP | 2020-014716 | A | 30 January 2020 | (Family: none) | |
| JP | 2011-251058 | A | 15 December 2011 | (Family: none) | |
| JP | 55-031595 | Y1 | 28 July 1980 | (Family: none) | |
| US | 4160402 | A | 10 July 1979 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020501853 A

**Non-patent literature cited in the description**

- *Multi-sensory Gamma Stimulation Ameliorates Alzheimer's-Associated Pathology and Improves Cognition Cell,* 04 April 2019, vol. 177 (2), 256-271.e22